# EUROPÄISCHE PATENTSCHRIFT

(11) **EP 2 269 728 B1**
(45) Veröffentlichungstag und Bekanntmachung des Hinweises auf die Patenterteilung: **31.01.2018**
(21) Anmeldenummer: 10164635.4
(22) Anmeldetag: 01.06.2010
(51) Int. Cl.: B01J 19/32, B01D 3/40

(54) **Verwendung von geordneten Packungen, die eine oder mehrere Anstaulagen, sowie ein oder mehrere Abscheidelagen aufweisen**
Use of ordered packages with one or more accumulation points and one or more separation points
Utilisation d'emballages ordonnés comportant une ou plusieurs positions d'empilement et une ou plusieurs positions de séparation

(30) Priorität: 09.06.2009 EP 09162248
(43) Veröffentlichungstag der Anmeldung: 05.01.2011
(73) Patentinhaber: BASF SE, 67056 Ludwigshafen am Rhein (DE)
(72) Erfinder: Kaibel, Gerd, 68623, Lampertheim (DE); Heida, Bernd, 67158, Ellerstadt (DE); Hugo, Randolf, 67246, Dirmstein (DE)
(74) Vertreter: BASF IP Association

(56) Entgegenhaltungen:
- EP-A1- 1 074 296
- WO-A1-2007/018516
- DE-A1- 4 238 716
- DE-A1- 10 105 660
- DE-C1- 10 019 196

## Beschreibung

### Beschreibung

Die Erfindung betrifft eine Verwendung von geordneten Packungen, die eine oder mehrere Anstaulagen sowie eine oder mehrere Abscheidelagen aufweisen.
Aus EP-A 1 074 296 ist eine geordnete Packung zum Wärme- und Stoffaustausch zwischen einer Flüssigkeit und einem Gas in einer Kolonne, mit mindestens einer Packungslage bekannt, die eine über ihre Höhe variierende, innere Geometrie aufweist, so dass sich durch eine geeignete Einstellung der Flüssigkeits- und Gasmengen in einem ersten, insbesondere unteren Bereich der Packungslage gezielt eine Sprudelschicht mit überwiegend disperser Gasphase und gleichzeitig in einem zweiten, insbesondere oberen Bereich der Packungslage, gezielt eine Filmströmung der Flüssigkeit mit überwiegend kontinuierlicher Gasphase ausbildet.
Eine derartige Packung stellt eine Übergangsform zwischen einer Packungskolonne mit überwiegend disperser Flüssigphase und einer Bodenkolonne mit überwiegend kontinuierlicher Flüssigphase dar und kombiniert günstige Eigenschaften einer Bodenkolonne, insbesondere hohe Stoffaustauschleistung in der Sprudelschicht mit günstigen Eigenschaften einer Packungskolonne, insbesondere Verhinderung von Tropfenmitriss und zusätzlichem Stoffaustausch an der Packungsoberfläche.
Insbesondere ist für Kolonnen, die mit derartigen Packungen bestückt sind, die Trennleistung gegenüber Kolonnen mit konventionellen geordneten Packungen wesentlich, um bis zu 60 %, höher. Die Trennleistung von Kolonnen wird üblicherweise als Anzahl der theoretischen Trennstufen je Meter Kolonnenhöhe (n_{th/m}) oder als Bauhöhe für eine theoretische Trennstufe (hight equivalent of a theoretical plate, hetp) definiert.

Für die destillative Auftrennung von Stoffgemischen aus Komponenten, deren Flüchtigkeiten sehr ähnlich sind, wird häufig eine Extraktivdestillation unter Zusatz eines selektiven Lösungsmittels durchgeführt. Hierbei handelt es sich um eine Substanz oder eine Mischung von Substanzen mit höherem Siedepunkt als die abzutragende Substanz. Das selektive Lösungsmittel als Zusatzstoff zur durch Extraktivdestillation aufzutrennenden Stoffmischung hat zur Folge, dass der Wirkungsgrad einer Trennstufe bei einer Extraktivdestillation nur etwa 15 bis 60 % beträgt, gegenüber etwa 70 bis 80 % bei klassischen Destillationen, ohne Zusatz eines selektiven Lösungsmittels.

Für Stofftrennungen durch Extraktivdestillation sind daher große Packungshöhen und entsprechend Kolonnen erforderlich.
Es war daher Aufgabe der Erfindung, die Trennleistung von Kolonnen zur Extraktivdestillation zu verbessern, bzw. die Höhe von Kolonnen zur Extraktivdestillation bei gleicher Trennaufgabe zu reduzieren.

Die Aufgabe wird gelöst durch die Verwendung gemäß Anspruch 1. Dem Verfahrenstechniker für destillative Trennaufgaben ist es bekannt, dass der Einsatz von geordneten Packungen in Verfahren zur Extraktivdestillation, die unter Zusatz eines hochsiedenden Lösungsmittels durchgeführt werden, kritisch ist, weil sie nach einer Verschmutzung schwierig zu reinigen sind und eine Ersatzbeschaffung zeit- und kostenaufwändig ist, weil geordnete Packungen immer maßgeschneidert sind.
Ein erhöhtes Fouling-Risiko besteht insbesondere bei der destillativen Auftrennung von C₄-Schnitten, das heißt Gemischen von Kohlenwasserstoffen, die überwiegend Kohlenwasserstoffe mit vier Kohlenstoffatomen pro Molekül enthalten, insbesondere aufgrund der Polymerisationsneigung von Komponenten mit konjugierten Doppelbindungen oder Dreifachbindungen. Auch viele selektive Lösungsmittel, die in Extraktivdestillationen eingestuft werden, tendieren zum Fouling.
Demgegenüber wurde überraschend gefunden, dass durch den erfindungsgemäßen Einsatz der speziellen geordneten Packung, mit über ihre Höhe variierender innerer Geometrie, die mindestens eine Anstaulage aufweist, in der der Flutpunkt erreicht oder überschritten wird, sowie mindestens eine Abscheidelage, in der der Flutpunkt nicht erreicht wird, in der oder den Anstaulagen ausreichend hohe Turbulenzen gewährleistet sind, damit das Fouling-Risiko bei Extraktivdestillationen. insbesondere bei Extraktivdestillationen von C₄-Schnitten, beherrschbar ist. Die Erklärung könnte in der reinigenden Wirkung verstärkter Turbulenzen in der Gas- und Flüssigphase liegen.
Erfindungsgemäß wird eine geordnete Packung eingesetzt, wie sie in EP-A 1 074 296 beschrieben ist, das heißt eine Packung mit mindestens einer Packungslage mit einem ersten, unteren und einem zweiten, oberen Ende, wobei die Packungslage eine über ihre Höhe variierende, innere Geometrie aufweist, so dass sich durch eine geeignete Einstellung der Flüssigkeits- und Gasmengen in einem ersten, insbesondere unteren Bereich der Packungslage gezielt eine Sprudelschicht mit überwiegend disperser Gasphase und gleichzeitig in einem zweiten, insbesodnere oberen Bereich der Packungslage gezielt eine Filmströmung der Flüssigkeit mit überwiegend kontinuierlicher Gasphase ausbildet.
Geordnete Packungen sind aus einer Vielzahl von Einzellagen aus Packungselementen, wie Blechen, Streckmetallen und Drahtgeweben, aufgebaut, die in einer regelmäßigen Struktur vertikal zueinander angeordnet sind und üblicherweise durch Befestigungsmittel wie Metalldrähte, dünne Metallstäbe oder Metallblechstreifen in einem Verbund zusammengehalten werden. Meist weisen diese Packungselemente selbst geometrische Strukturierung auf, beispielsweise in Form von Knicken oder kreisförmigen Löchern mit etwa 4 bis 6 mm Durchmesser. Die Öffnungen dienen dazu, die Flutgrenze der Packung anzuheben und eine höhere Kolonnenbelastung zu ermöglichen.
Die beschriebenen hydrodynamischen Betriebszustände können dadurch erreicht werden, dass der Strömungswiderstand über die Höhe der Packungslage variiert. Bevorzugt weist der erste, gegebenenfalls untere Bereich der Packungslage einen größeren Strömungswiderstand auf als der zweite, gegebenenfalls obere Bereich der Packungslage.
Der erste Bereich der Packungslage befindet sich bevorzugt in einem unteren Bereich der Packungslage und der zweite Bereich der Packungslage bevorzugt in einem oberen Bereich der Packungslage. Im Rahmen der vorliegenden Erfindung erstrecken sich der erste, gegebenenfalls untere Bereich und der zweite, gegebenenfalls obere Bereich der Packungslage bevorzugt über die gesamte Querschnittsfläche der Packungslage. Der erste, untere Bereich der Packungslage kann direkt an das untere Ende der Packungslage und der zweite, obere Bereich der Packungslage direkt an das obere Ende der Packungslage angrenzen. In einer bevorzugten Ausführungsform schließt sich der erste, gegebenenfalls untere Bereich der Packungslage direkt an den zweiten, gegebenenfalls oberen Bereich an.
Die geordneten Packungen sind in vorteilhafter Weise Blech- oder Gewebepackungen, die aus ebenen Blechen oder Gewebelagen gebildet sind, die in Kreuzkanalstruktur geformt sind.
Insbesondere weisen die die Kreuzkanalstruktur bildenden ebenen Bleche oder Gewebelagen Knicke auf, die gegen der Horizontalen in der einen oder in den mehreren Abscheidelagen einen Winkel von < 60°, bevorzugt einen Winkel von 45°, ausbilden.

Die Blech- oder Gewebepackung in Kreuzkanalstruktur ist bevorzugt dergestalt ausgebildet ist, dass über die Höhe einer Abscheidelage jeweils 2 bis 10, bevorzugt 2 bis 4 Kreuzungspunkte der Knicke vorhanden sind.
Die Höhe der einen oder der mehreren Abscheidelagen beträgt jeweils 5 bis 50 cm, bevorzugt jeweils 10 bis 20 cm.
Für die erfindungsgemäßen Verwendungen werden als selektives Lösungsmittel eine oder mehrere Substanzen eingesetzt, die eine Erhöhung der relativen Flüchtigkeit der abzutrennenden Substanz (d.h. des Wertproduktes) gegenüber den übrigen Komponenten des diese enthaltenden Gemisches bewirken. In der Regel handelt es sich hierbei um hochsiedende Substanzen, das heißt um Substanzen, deren Siedepunkt gegenüber dem aufzutrennenden Gemisch höher ist. Besonders bevorzugt werden als selektive Lösungsmittel hochsiedende polare Substanzen eingesetzt, insbesondere ausgewählt aus N-Methylpyrrolidon, Dimethylformamid und Acetonitril, oder Mischungen hiervon.
Bevorzugt können dem selektiven Lösungsmittel ein oder mehrere Zusatzstoffe zugegeben werden, insbesondere Wasser und/oder ein oder mehrere C₁- bis C₄-Alkohole. Als C₁- bis C₄-Alkohole können Monohydroxyalkohole oder Diole eingesetzt werden. Bevorzugt sind Ethanol und n- oder Isopropanol.
Bevorzugt kann die Extraktivdestillation in Gegenwart eines oder mehrerer gegenüber der abzutrennenden Substanz höher siedenden Stabilisators durchgeführt werden, um die Polymerisationsanfälligkeit weiter zu begrenzen.

Gegenstand der Erfindung ist die Verwendung einer geordneten Packung mit einer oder mehreren Anstaulagen und einer oder mehreren Abscheidelagen in einem Verfahren zur Auftrennung von 1,3-Butadien aus einem C₄-Schnitt durch Extraktivdestillation und/oder die Auftrennung von Butanen und Butenen aus einem C₄-Schnitt.

Die Erfindung ist nicht eingeschränkt bezüglich der konkreten Verfahrensführung zur Abtrennung von 1,3-Butadien aus einem C₄-Schnitt.
Bevorzugt können erfindungsgemäß geordnete Packungen in einer Kolonne zur Gewinnung von 1,3-Butadien durch Extraktivdestillation eingesetzt werden, wie sie in DE 101 05 660 beschrieben ist, das heißt in einem Verfahren wonach die Auftrennung des C₄-Schnittes in einer Trennwandkolonne mit bis zum oberen Ende der Trennwandkolonne durchgezogene Trennwand und einer der Trennwandkolonne vorgeschalteten Extraktivwaschkolonne durchgeführt wird.
Weiter bevorzugt wird die geordnete Packung mit über ihrer Höhe variierende innere Geometrie in einem Verfahren zur Abtrennung von 1,3-Butadien aus einem C₄-Schnitt durch Extraktivdestillation eingesetzt, wie es in DE 103 22 655 beschrieben ist.
Danach wird für die Extraktivdestillation eine Trennwandkolonne eingesetzt, in der eine Trennwand in Kolonnenlängsrichtung unter Ausbildung eines ersten Teilbereichs, eines zweiten Teilbereichs und eines unteren gemeinsamen Kolonnenbereichs angeordnet ist, die bis zum oberen Ende der Kolonne durchgezogen ist, und die mit einer vorgeschalteten Extraktivwaschkolonne verbunden ist, eingesetzt.
Bevorzugt wird
- der C₄-Schnitt dem ersten Teilbereich der Trennwandkolonne, bevorzugt in dessen mittleren Bereich, zugeführt,
- der Kopfstrom aus dem ersten Teilbereich der Trennwandkolonne der Extraktivwaschkolonne, in deren unteren Bereich, zugeführt,
- in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
- die im selektiven Lösungsmittel weniger als 1,3-Butadien löslichen Komponenten des C₄-Schnittes werden über Kopf der Extraktivwaschkolonne abgezogen,
- der Sumpfstrom aus der Extraktivwaschkolonne in den oberen Bereich des ersten Teilbereichs der Trennwandkolonne zurückgeführt,
- der Trennwandkolonne im oberen Bereich des zweiten Teilbereichs ein zweiter Teilstrom des selektiven Lösungsmittels zugeführt,
das Kopfprodukt aus dem zweiten Teilbereich (B) der Trennwandkolonne als Roh-1,3-Butadien abgezogen und
aus dem unteren gemeinsamen Kolonnenbereich der Trennwandkolonne ein Sumpfstrom bestehend aus gereinigtem Lösungsmittel abgezogen wird, der in das Verfahren recycliert wird.

Bevorzugt wird somit der aufzutrennende C₄-Schnitt dem ersten Teilbereich der Trennwandkolonne, besonders bevorzugt in dessen mittleren Bereich, zugeführt;
der Kopfstrom aus dem ersten Teilbereich der Trennwandkolonne wird der vorgeschalteten Extraktivwaschkolonne in deren unteren Bereich zugeführt,
in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
die im selektiven Lösungsmittel weniger als 1,3-Butadien-löslichen Komponenten des C₄-Schnittes über Kopf der Extraktivwaschkolonne abgezogen, besonders bevorzugt in einem Kondensator am Kopf der Extraktivwaschkolonne kondensiert und teilweise als Rücklauf erneut auf die Extraktivwaschkolonne aufgegeben, im übrigen als ein überwiegend Butane und Butene enthaltendes Nebenprodukt, häufig als Raffinat 1 bezeichnet, abgezogen.

Durch die Zuführung des Sumpfstroms der Extraktivwaschkolonne, d.h. einem Strom, der neben dem selektiven Lösungsmittel 1,3-Butadien, Butane, Butene sowie die besser als 1,3-Butadien im selektiven Lösungsmittel löslichen Komponenten des C₄-Schnitts enthält, in den oberen Bereich des ersten Teilbereichs der Trennwandkolonne kann durch den Stoffaustausch zwischen diesem Strom und dem dampfförmig aufgegebenen C₄-Schnitt im oberen Bereich des ersten Teilbereichs der Trennwandkolonne eine Gegenstromextraktion unter Ausschleusung der im selektiven Lösungsmitttel weniger als das 1,3-Butadien löslichen Komponenten am Kopf des ersten Teilbereichs der Trennwandkolonne erfolgen.

Am unteren Ende der Trennwand fällt ein dampfförmiger Strom an, der neben 1,3-Butadien die im selektiven Lösungsmittel besser als 1,3-Butadien-löslichen Komponenten des C₄-Schnitts, insbesondere C₄-Acetylene, enthält. Diese werden aus dem aufsteigenden dampfförmigen Strom im Gegenstrom mit einem zweiten Teilstrom des selektiven Lösungsmittels, das im oberen Bereich des zweiten Teilbereichs der Trennwandkolonne aufgegeben wird, ausgewaschen. Das dampfförmige Kopfprodukt aus dem zweiten Teilbereich der Trennwandkolonne wird abgezogen, wobei es bevorzugt in einem Kondensator am Kolonnenkopf kondensiert, ein Teilstrom des kondensierten Kopfstromes zurück auf den Teilbereich B der Trennwandkolonne als Rücklauf gegeben und der kondensierte Kopfstrom wird im übrigen als Roh-1,3-Butadien abgezogen wird.
Im unteren gemeinsamen Kolonnenbereich erfolgt eine vollständige Entgasung des Lösungsmittels, wobei am Sumpf der Extraktivdestillationskolonne ein gereinigtes Lösungsmittel erhalten wird.

Bei der Bestimmung des hierfür erforderlichen Energieeintrags über den Sumpfverdampfer der Extraktivdestillationskolonne wird der Verfahrenstechniker die thermische Belastbarkeit der Substanz oder des Substanzgemisches berücksichtigen, die jeweils im konkreten Fall als selektives Lösungsmittel eingesetzt wurden.

In einer weiteren bevorzugten Ausführungsform wird die Extraktivdestillation zur Abtrennung von 1,3-Butadien aus einem C₄-Schnitt ebenfalls in einer Anlage mit einer Trennwandkolonne mit bis zum oberen Ende der Kolonne durchgezogener Trennwand, die mit einer vorgeschalteten Extraktivwaschkolonne verbunden ist, durchgeführt, wobei jedoch der aufzutrennende C₄-Schnitt, abweichend von der vorstehend dargelegten Ausführungsform, nicht dem ersten Teilbereich der Trennwandkolonne, sondern der vorgeschalteten Extraktivwaschkolonne, in deren unteren Bereich, zugeführt wird.
Es ist nicht zwingend erforderlich, die Gewinnung des Nebenprodukts aus Butanen und Butenen, des sogenannten Raffinats 1, in einer von der Extraktivdestillationskolonne getrennten, vorgeschalteten Extraktivwaschkolonne vorzusehen. Es ist auch möglich, die Extraktivwaschkolonne in den ersten Teilbereich der als Extraktivdestillationskolonne eingesetzten Trennwandkolonne zu integrieren, sofern es technisch und wirtschaftlich unter Berücksichtigung der konkreten Rahmenbedingungen für das Verfahren, insbesondere der Zusammensetzung des aufzutrennenden C₄-Schnitts und der Spezifikation für Raffinat 1 realisierbar ist, die Zahl der theoretischen Trennstufen im ersten Teilbereich der Trennwandkolonne entsprechend zu erhöhen.
Die erfindungsgemäße Verwendung von geordneten Packungen, die Anstaulagen aufweisen, wird in Verfahren zur Auftrennung eines C₄-Schnittes durch Extraktivdestillation mit einem selektiven Lösungsmittel eingesetzt wonach 1,3-Butadien, Butene und Butane als jeweils getrennte Ströme erhalten werden.
Die Erfindung ist nicht eingeschränkt bezüglich der konkreten Verfahrensführung zur Auftrennung von C₄-Schnitten in 1,3-Butadien, Butane und Butene.
Bevorzugt ist die Verwendung in einem Verfahren entsprechend der WO2004/011406, wonach eine Extraktivdestillation in einer Kolonne durchgeführt wird, die eine Längsrichtung derselben angeordnete Trennwand aufweist, die bis zum oberen Ende der Kolonne durchgezogen ist, und entsprechend einen ersten oberen Teilbereich, einen zweiten oberen Teilbereich sowie einen unteren gemeinsamen Kolonnenbereich ausbildet, und wobei aus dem ersten oberen Teilbereich ein die Butane umfassender Kopfstrom, aus dem zweiten oberen Teilbereich ein die Butene umfassender Kopfstrom, und aus dem unteren gemeinsamen Kolonnenbereich ein die Kohlenwasserstoffe aus dem C₄-Schnitt umfassender Strom abgetrennt wird, die im selektiven Lösungsmittel besser löslich sind als die Butane und die Butene.

Bevorzugt kann der aus der Trennwandkolonne mit bis zum oberen Ende derselben vollständig durchgezogener Trennwand abgezogene Strom, der die im selektiven Lösungsmittel besser als die Butane und Butene löslichen Kohlenwasserstoffe umfasst, einer ersten Destillationskolonne zugeführt werden, worin er in einem Kopfstrom aufgetrennt wird, umfassend 1,3-Butadien, Propin, gegebenenfalls weitere Leichtsieder und gegebenenfalls Wasser sowie in einem Sumpfstrom, umfassend 1,3-Butadien, 1,2-Butadien, Acetylene sowie gegebenenfalls weitere Schwersieder, wobei der Anteil des Butadiens im Sumpfstrom der ersten Destillationskolonne dergestalt geregelt wird, dass er mindestens so hoch ist, dass er die Acetylene außerhalb des selbstzersetzungsgefährdeten Bereiches verdünnt und dass der Kopfstrom der ersten Destillationskolonne in einer zweiten Destillationskolonne zugeführt und in der zweiten Destillationskolonne in einen Kopfstrom, umfassend Propin, gegebenenfalls weitere Leichtsieder und gegebenenfalls Wasser sowie in einen Sumpfstrom, umfassend Rein-1,3-Butadien aufgetrennt wird.
Gegenstand der Erfindung ist auch die Verwendung einer geordneten Packung mit einer oder mehreren Anstaulagen und einer oder mehreren Abscheidelagen in einem Verfahren zur Auftrennung eines Gemisches durch Extraktivdestillation, wobei die durch Extraktivdestillation abzutrennende Substanz Isopren ist.
Die Erfindung wird im Folgenden anhand einer Zeichnung näher erläutert.
Es zeigen im Einzelnen:
- Figur 1: eine Packungslage einer Ausführungsform einer geordneten Packung zur erfindungsgemäßen Verwendung,
- Figur 2: hintereinander angeordnete Packungselemente einer Packungslage einer weiteren Ausführungsform einer geordneten Packung zur erfindungsgemäßen Verwendung und
- Figur 3: hintereinander angeordnete Packungselemente einer Packungslage einer weiteren Ausführungsform einer geordneten Packung zur erfindungsgemäßen Verwendung.

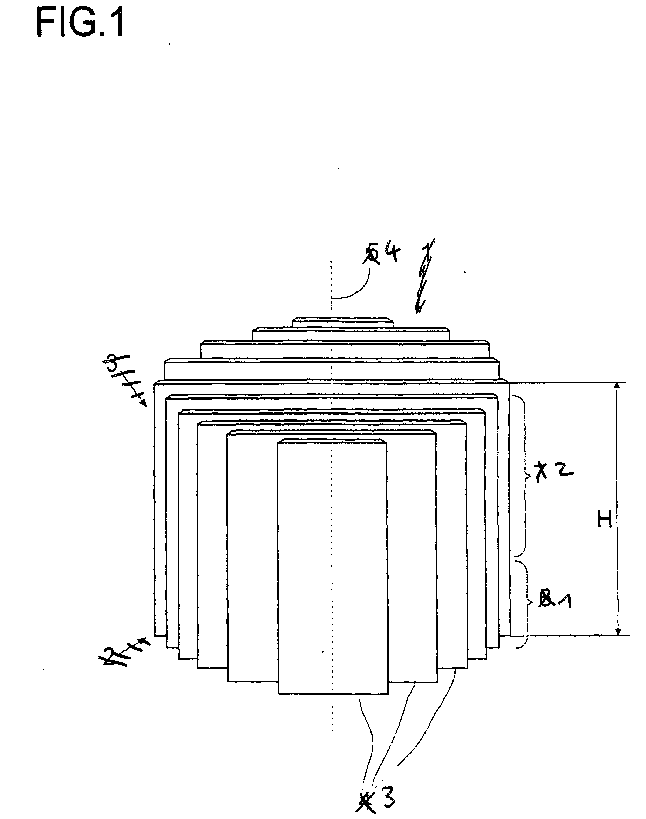

In Figur 1 ist eine Ausführungsform einer geordneten Packung zur Verwendung gemäß der vorliegenden Erfindung dargestellt. Die Packungslage 1 weist ein erstes, unteres Ende und ein zweites, oberes Ende auf. Sie besitzt eine Höhe H von beispielsweise 0,2 m. Die Packung weist sich berührende, flächige Packungselemente 4 aus mit Knicken (nicht dargestellt) versehenen ebenen Blechen 3 auf. Die Bezugsziffer 4 zeigt die Längsachse der Packung. Die Packung hat einen kreisförmigen Querschnitt. Die innere Geometrie der Packung variiert über deren Höhe (nicht dargestellt). Die Packung weist eine erste, untere Anstaulage 1 auf, deren innere Geometrie sich von einer zweiten, oberen Abscheidelage 2 unterscheidet. Die Anstaulage 1 weist einen größeren Strömungswiderstand auf als die Abscheidelage 2. Durch eine geeignete Einstellung der Flüssigkeits- und Gasmengen bildet sich in der Anstaulage 1 eine Sprudelschicht mit überwiegend disperser Gasphase und gleichzeitig in der Abscheidelage 2 eine Filmströmung der Flüssigkeit mit überwiegend kontinuierlicher Gasphase aus. Die Anstaulage 1 und die Abscheidelage 2 erstrecken sich über die gesamte Querschnittsfläche der Packung.

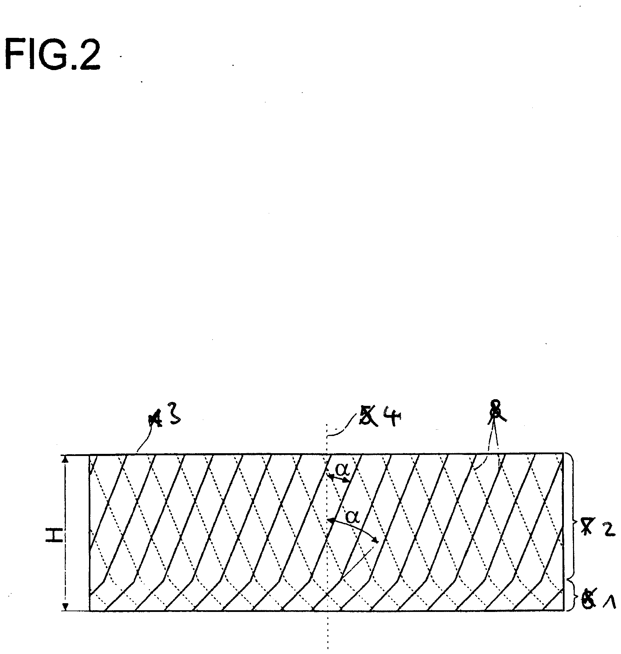

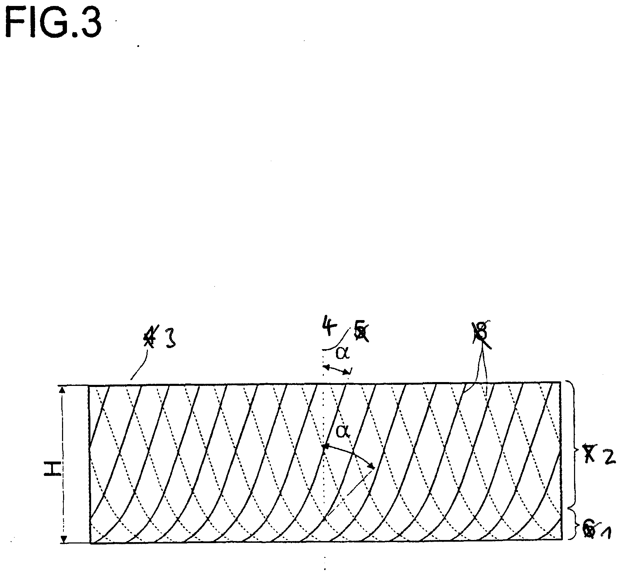

In den Figuren 2 und 3 sind jeweils hintereinander angeordnete ebene Bleche 3 verschiedener Ausführungsformen der geordneten Packung zur Verwendung gemäß der vorliegenden Erfindung schematisch dargestellt. Die durchgezogenen Linien zeigen die Knickverläufe des ersten, dritten, fünften, usw. ebenen Bleches 3 und die gestrichelten Linien die Knickverläufe des zweiten, vierten, sechsten usw. ebenen Bleches 3.

## Patentansprüche

1. Verwendung von geordneten Packungen, die eine oder mehrere Anstaulagen (1) aufweisen, deren spezifische Oberfläche so gewählt wird, dass in der einen oder den mehreren Anstaulagen (1) der Flutpunkt erreicht oder überschritten wird, sowie eine oder mehrere Abscheidelagen (2), deren spezifische Oberfläche so gewählt wird, dass der Flutpunkt nicht erreicht wird als trennwirksame Einbauten in einer Kolonne zur Durchführung eines Verfahrens zur destillativen Abtrennung durch Extraktivdestillation unter Zusatz eines selektiven Lösungsmittels von 1,3-Butadien oder einer überwiegend Butane und einer überwiegend Butene enthaltende Fraktion aus einem C4-Schnitt oder von Isopren aus einem Isopren enthaltenden Gemisch.

2. Verwendung nach Anspruch 1, **dadurch gekennzeichnet, dass** die spezifische Oberfläche der einen oder der mehreren Anstaulagen (1) dergestalt gewählt wird, dass der Flutpunkt in der einen oder in den mehreren Anstaulagen (1) um 10 bis 100 %, bevorzugt um 20 bis 50 % überschritten wird, und dass die spezifische Oberfläche der einen oder der mehreren Abscheidelagen (2) dergestalt gewählt wird, dass der Flutpunkt in der einen oder in den mehreren Abscheidelagen (2) um 20 bis 80 %, bevorzugt um 30 bis 50 %, unterschritten wird.

3. Verwendung nach Anspruch 1 oder 2, **dadurch gekennzeichnet, dass** die geordneten Packungen Blech- oder Gewebepackungen sind, die aus ebenen Blechen (3) oder Gewebelagen gebildet sind, die in Kreuzkanalstruktur geformt sind.

4. Verwendung nach Anspruch 3, **dadurch gekennzeichnet, dass** die die Kreuzkanalstruktur bildenden ebenen Bleche oder Gewebelagen Knicke aufweisen, die gegen der Horizontalen in der einen oder in den mehreren Abscheidelagen (1) einen Winkel von < 60°, bevorzugt einen Winkel von 45°, ausbilden.

5. Verwendung nach Anspruch 3 oder 4, **dadurch gekennzeichnet, dass** die Blech- oder Gewebepackung in Kreuzkanalstruktur dergestalt ausgebildet ist, dass über die Höhe einer Abscheidelage (1) jeweils 2 bis 10, bevorzugt 2 bis 4 Kreuzungspunkte der Knicke vorhanden sind.

6. Verwendung nach einem der Ansprüche 1 bis 5, **dadurch gekennzeichnet, dass** die Höhe der einen oder der mehreren Abscheidelagen jeweils 5 bis 50 cm, bevorzugt jeweils 10 bis 20 cm, beträgt.

7. Verwendung nach einem der Ansprüche 1 bis 6, **dadurch gekennzeichnet, dass** die Extraktivdestillation in Gegenwart eines oder mehrerer gegenüber der abzutrennenden Substanz höher siedenden Stabilisators durchgeführt wird.

8. Verwendung nach einem der Ansprüche 1 bis 7, **dadurch gekennzeichnet, dass** als selektives Lösungsmittel eine oder mehrere gegenüber der abzutrennenden Substanz hochsiedende polare Substanzen, bevorzugt ausgewählt aus N-Methylpyrrolidon, Dimethylformamid und Acetonitril, oder Mischungen hiervon mit oder ohne Wasserzugabe, eingesetzt werden.

9. Verwendung nach Anspruch 8, **dadurch gekennzeichnet, dass** dem selektiven Lösungsmittel Zusatzstoffe zugegeben werden.

10. Verwendung nach Anspruch 9, **dadurch gekennzeichnet, dass** als Zusatzstoffe Wasser und/oder ein C₁- bis C₄-Alkohol zugegeben wird.

11. Verwendung nach Anspruch 10, **dadurch gekennzeichnet, dass** als C₁- bis C₄-Alkohol Ethanol, n-Propanol oder Isopropanol eingesetzt wird.

## Claims

1. The use of structured packings which have one or more backup layers (1), whose specific surface area is chosen so that the flooding point is reached or exceeded in the one or more backup layers (1), and one or more separation layers (2), whose specific surface area is chosen so that the flooding point is not reached, as internals having a separating effect in a column for carrying out a method for the distillative separation by extractive distillation with addition of a selective solvent of 1,3-butadiene or a fraction predominantly comprising butanes and a fraction predominantly comprising butenes from a C4 cut or of isoprene from a mixture comprising isoprene.

2. The use according to claim 1, wherein the specific surface area of the one or more backup layers (1) is chosen so that the flooding point in the one or more backup layers (1) is exceeded by from 10 to 100%, preferably by from 20 to 50%, and in that the specific surface area of the one or more separation layers (2) is chosen so that the flooding point in the one or more separation layers (2) is fallen short of by from 20 to 80%, preferably by from 30 to 50%.

3. The use according to claim 1 or 2, wherein the structured packings are sheet-metal or fabric packings which are formed from flat metal sheets (3) or fabric layers which have a cross-channel structure.

4. The use according to claim 3, wherein the flat metal sheets or fabric layers forming the cross-channel structure have bends which make an angle of <60°, preferably an angle of 45°, relative to the horizontal in the one or more separation layers (1).

5. The use according to claim 3 or 4, wherein the sheet-metal or fabric packing is formed with a cross-channel structure in such a way that in each case from 2 to 10, preferably from 2 to 4, intersection points of the bends are present over the height of a separation layer (1).

6. The use according to any of claims 1 to 5, wherein the height of the one or more separation layers is in each case from 5 to 50 cm, preferably in each case from 10 to 20 cm.

7. The use according to any of claims 1 to 6, wherein the extractive distillation is carried out in the presence of one or more stabilizers having a boiling point which is higher than that of the substance to be separated off.

8. The use according to any of claims 1 to 7, wherein one or more polar substances having a high boiling point compared with the substance to be separated off, preferably selected from N-methylpyrrolidone, dimethylformamide and acetonitrile, or mixtures thereof, with or without addition of water, are used as the selective solvent.

9. The use according to claim 8, wherein additives are added to the selective solvent.

10. The use according to claim 9, wherein the additives added are water and/or C₁- to C₄-alcohol.

11. The use according to claim 10, wherein the C₁- to C₄-alcohol used is ethanol, n-propanol or isopropanol.

## Revendications

1. Utilisation de garnissages ordonnés, qui comprennent une ou plusieurs couches d'accumulation (1), dont la surface spécifique est choisie de sorte que le point d'engorgement soit atteint ou dépassé dans la ou les couches d'accumulation (1), ainsi qu'une ou plusieurs couches de séparation (2), dont la surface spécifique est choisie de sorte que le point d'engorgement ne soit pas atteint, en tant que composants internes à effet de séparation dans une colonne pour la réalisation d'un procédé de séparation distillative par distillation extractive avec ajout d'un solvant sélectif de 1,3-butadiène ou d'une fraction contenant principalement des butanes ou contenant principalement des butènes à partir d'une coupe en C4 ou d'isoprène à partir d'un mélange contenant de l'isoprène.

2. Utilisation selon la revendication 1, **caractérisée en ce que** la surface spécifique de la ou des couches d'accumulation (1) est choisie de sorte que le point d'engorgement soit dépassé de 10 à 100 %, de préférence de 20 à 50 %, dans la ou les couches d'accumulation (1), et **en ce que** la surface spécifique de la ou des couches de séparation (2) est choisie de manière à rester inférieur de 20 à 80 %, de préférence de 30 à 50 %, au point d'engorgement.

3. Utilisation selon la revendication 1 ou 2, **caractérisée en ce que** les garnissages ordonnés sont des garnissages en tôle ou en tissu, qui sont formés à partir de tôles plates (3) ou de couches de tissu, qui sont mises sous la forme d'une structure de canaux croisés.

4. Utilisation selon la revendication 3, **caractérisée en ce que** les tôles plates ou les couches de tissu formant la structure de canaux croisés présentent des plis, qui forment un angle < 60°, de préférence un angle de 45°, par rapport à l'horizontale, dans la ou les couches de séparation (1).

5. Utilisation selon la revendication 3 ou 4, **caractérisée en ce que** le garnissage en tôle ou en tissu est configuré selon une structure de canaux croisés de sorte que 2 à 10, de préférence 2 à 4, points de croisement des plis soient présents à chaque fois sur la hauteur d'une couche de séparation (1).

6. Utilisation selon l'une quelconque des revendications 1 à 5, **caractérisée en ce que** la hauteur de la ou des couches de séparation est à chaque fois de 5 à 50 cm, de préférence à chaque fois de 10 à 20 cm.

7. Utilisation selon l'une quelconque des revendications 1 à 6, **caractérisée en ce que** la distillation extractive est réalisée en présence d'un ou de plusieurs stabilisateurs ayant un point d'ébullition plus élevé que la substance à séparer.

8. Utilisation selon l'une quelconque des revendications 1 à 7, **caractérisée en ce qu'**une ou plusieurs substances polaires ayant un point d'ébullition élevé par rapport à la substance à séparer, de préférence choisies parmi la N-méthylpyrrolidone, le diméthylformamide et l'acétonitrile ou leurs mélanges avec ou sans ajout d'eau, sont utilisées en tant que solvant sélectif.

9. Utilisation selon la revendication 8, **caractérisée en ce que** des additifs sont ajoutés au solvant sélectif.

10. Utilisation selon la revendication 9, **caractérisée en ce que** de l'eau et/ou un alcool en C₁ à C₄ sont ajoutés en tant qu'additifs.

11. Utilisation selon la revendication 10, **caractérisée en ce que** de l'éthanol, du n-propanol ou de l'isopropanol est utilisé en tant qu'alcool en C₁ à C₄.
